# EUROPEAN PATENT APPLICATION

(11) **EP 3 800 245 A1**
(43) Date of publication of application: **07.04.2021**
(21) Application number: 19201489.2
(22) Date of filing: 04.10.2019
(51) Int. Cl.: C12N 5/073, C12N 15/113

(54) **EMBRYO TRANSFER MEDIUM**

(71) Applicant: Academisch Medisch Centrum, 1105 AZ Amsterdam (NL)
(72) Inventor: The designation of the inventor has not yet been filed
(74) Representative: de Pauw, Elmar Sebastian David

(57) **Abstract**

The invention provides mir320a or a mimic thereof for use in an assisted reproduction treatment. The invention further provides an embryo transfer medium suitable for use in a human comprising mir320a or a mimic thereof.

## Description

### FIELD OF THE INVENTION

The present invention relates to embryo transfer media that provide an increased migration of decidualized human endometrial stromal cells (hESCs) and subsequent improved implantation in assisted reproduction therapies and to the use of hsa-miR-320a in assisted reproduction treatments.

### BACKGROUND OF THE INVENTION

Implantation of an embryo in the uterus in human is characterized by its inefficiency and relatively low success rate. In natural conception, live birth rates per cycle range from 30-40% with a miscarriage rate of 10%, suggesting that all residual embryos are lost before or right after implantation (Wilcox et al. 1988; Zinaman et al. 1996; Macklon 2002). Despite advances in reproductive technologies, implantation failure remains a major limiting factor in improving pregnancy rates in in vitro fertilization (IVF)/ Intracytoplasmic sperm injection (ICSI).

It is an objective of the invention to improve pregnancy rates in in vitro fertilization (IVF)/ Intracytoplasmic sperm injection (ICSI).

### SUMMARY OF THE INVENTION

The present invention is based on the surprising finding that human preimplantation embryos of high morphological quality specifically secrete factors that improve migration of human endometrial stromal cells (hESCs). Of these factors, miRNA hsa-miR-320a alone appeared to be sufficient to induce this migration phenotype. By artificially mimicking endogenous miRNA function through transfection of hESCs with miRNA320a mimics, the inventors herein show that hsa-miR-320a stimulates migration of decidualized hESCs, accurately resembling the response that is typically triggered by high-quality embryos. Furthermore, they found that specific inhibition of hsa-miR-320a in decidualized hESCs neutralizes the pro-migratory effect of high-quality embryos. Additionally, they found that hsa-miR-320a alters gene expression patterns in decidualized hESCs involved in cell adhesion, and cytoskeleton organization. In contrast, low-quality embryos secrete a different subset of miRNAs, namely hsa-miR-19a-3p and hsa-miR-19b-3p, that do not affect decidualized hESCs migration. In conclusion, they found that a soluble human miRNA, hsa-miR-320a, is specifically secreted by high-quality human preimplantation embryos, and directly stimulates migration of decidualized hESCs. Together, these results indicate that hsa-miR-320a directly promotes embryo implantation and can therefore increase success rates in assisted conception such as IVF and ICSI.

In Feng et al. (SCIENTIFIC REPORTS |5 8689| DOI: 10.1038/srep08689), it was disclosed that miR-320 and miR-197 in human follicular fluid are associated with oocyte quality and subsequent embryonic development potential. They observed that knocking down miR-320 in mouse oocytes negatively affected embryonic developmental potential by inhibiting expression of Wnt signaling pathway. They observed that most of the miR-320 inhibitor-injected oocytes arrested after fertilization at the 2-cell stage and only a few proceeded to develop into blastocysts, indicating that the presence of miR-320 in oocytes is essential for subsequent embryonic development at a very early developmental stage. However, Feng at al do not describe that, at the time of implantation in the uterus, embryos secrete miR-320a that promote endometrial cell migration, nor do they describe that miR-320a alone can mimic this effect or any effect on hESCs. Furthermore, there is no clear link between embryo-derived miRNAs and a possible biological mechanism of action during implantation. There is no suggestion in Feng et al. that addition of miR-320a might result in any response on human endometrial stromal cells let alone improve the success rate in assisted reproduction. Therefore, the invention provides mir320a for use in an assisted reproduction treatment.

The invention further provides an embryo transfer medium suitable for use in a human comprising mir320a or a mimic thereof.

In Wang et al. ONCOLOGY LETTERS 13: 3247-3252, 2017, a DMEM culture medium comprising miR-320a has been disclosed, but this is not an embryo transfer media suitable for human use, due to the presence of for instance fetal bovine serum proteins.

In a preferred embodiment, the embryo transfer medium according to the invention comprises a medium approved for use in human IVF treatments.

Preferably, the embryo transfer medium according to the invention comprises 10-50, more preferably about 25 nM mir320a or miRNA mimic.

The invention further provides the embryo transfer medium according to the invention for use in an assisted reproduction treatment.

### BRIEF DESCRIPTION OF THE FIGURES

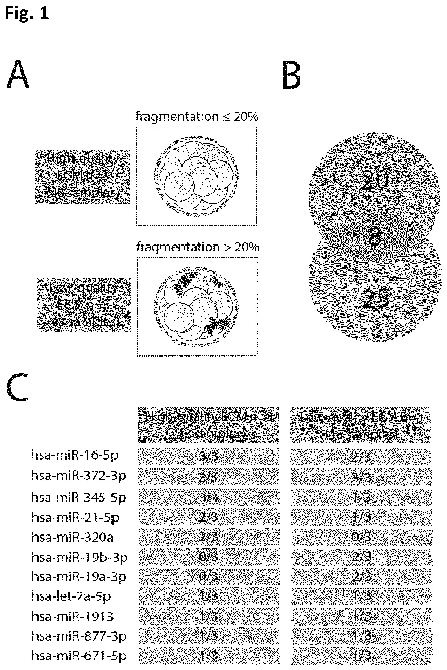

Figure 1 shows that high- and low-quality human preimplantation embryos secrete specific subsets of miRNAs as measured in embryo conditioned medium (ECM). (A) For both high- and low-quality embryos, 48 individually cultured samples were collected that were pooled per 16 to create 3 biological replicates. (B) High-quality embryos secreted 20 unique miRNAs, low-quality embryos secreted 25 unique miRNAs and 8 miRNAs were overlapping. (C) The most frequently expressed miRNAs were present in both high- and low-quality ECM, except for hsa-miR-320a which is high-quality ECM specific, and hsa-miR-19a-3p and hsa-miR-19b-3p which are low-quality ECM specific. ECM, embryo conditioned medium.

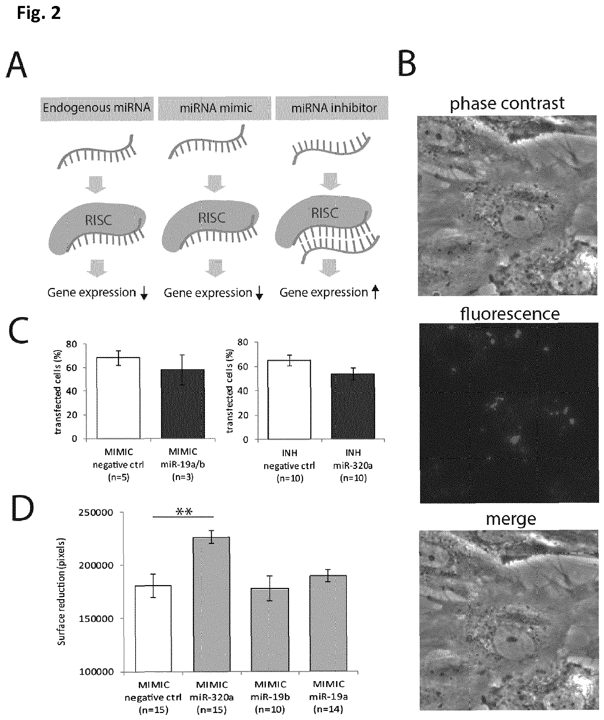

Figure 2 shows that Hsa-miR-320a mimics high-quality ECM-induced migration of decidualized hESCs. (A) Endogenous miRNA are assembled into RISC and suppress expression of target genes; miRNA mimics are likewise assembled into RISC and suppress expression of target genes; miRNA inhibitors bind to complementary mature miRNA and thereby inhibit miRNA function. (B-C) Transfection percentages of miRNA mimics and inhibitors into decidualized hESCs is not significantly different compared to negative control mimics or inhibitors. (D) Mimics for hsa-miR-320a stimulated migration of decidualized hESCs whereas all other mimics had no effect. hESCs, human endometrial stromal cells. Data are expressed as mean ± sem. Values that are significantly different are labelled by asterisks (*p<0.05).

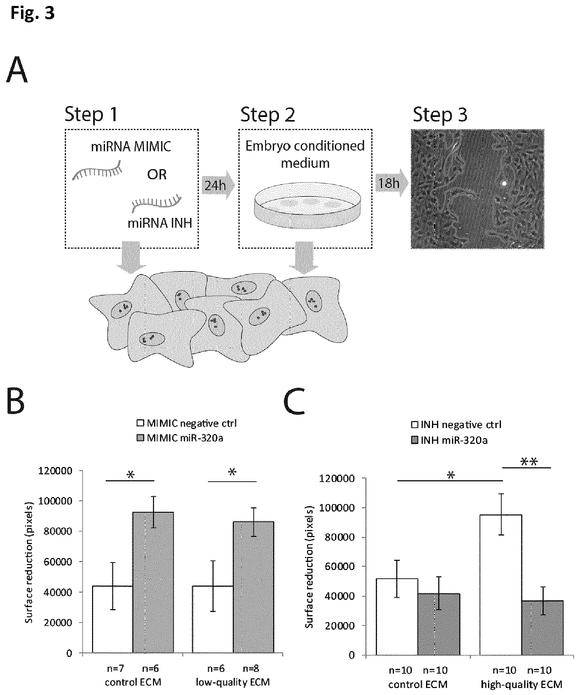

Figure 3 shows that Hsa-miR-320a mimics and inhibitors interfere with ECM to regulate migration of decidualized hESCs. (A) Decidualized hESCs were first transfected with miRNA mimics or inhibitors for 24 hours, after which migration assays (18 hours) were performed during co-culture with ECM. (B) Mimics for hsa-miR-320a stimulated decidualized hESCs migration when co-cultured with control ECM or low-quality ECM. (C) Inhibitors for hsa-miR-320a neutralized the pro-migratory effect of high-quality ECM. hESCs, human endometrial stromal cells. Data are expressed as mean ± sem. Values that are significantly different are labelled by asterisks (*p<0.05).

### DETAILED DESCRIPTION OF THE INVENTION

As used herein, the term "mimic" of a miRNA means a single-stranded or double-stranded oligonucleotide with the same or substantially similar-base composition and sequence (including chemically modified bases) as a particular natural miRNA and which is designed to mimic the activity of such miRNA.

As used herein, the term "assisted reproduction treatment" refers to any method which involves the creation of an embryo through the use of medical procedures, including IVF and ICSI.

In vitro fertilization (IVF) is the process of fertilization of an egg (oocyte) with sperm outside of the body of the mother in a laboratory dish. The fertilized egg, or embryo, is usually cultured in vitro for some period of time or until a desired developmental milestone is reached. For example, some embryos are cultured until the embryo reaches 6-8 cells, or until reaching the blastocyst stage. Other embryos may be cultured for a period of time such as 5 or 6 days, for example. After the desired culturing has been accomplished, embryo selection and embryo transfer occur by which an embryo is selected and transferred into the uterus of a female patient, and/or in some cases preserved (e.g., cryopreservation) for later implantation.

All miRNAs mention herein are known per se and sequences of them are publicly available from the data base.

The terms "mir320a", "hsa-mir-320", "hsa-mir-320a"and "mir320" are used interchangeably herein and refer to any mature miR320a sequence derived from the hsa-mir-320a pre miRNA sequence CUCCCCUCCGCCUUCUCUUCCCGGUUCUUCCCGGAGUCGGGAAAAGCUGGGUUGAGAGGGCGAAAAAGG AUG (SEQ ID NO:1), including AAAAGCUGGGUUGAGAGGGCGA (SEQ ID NO:2, hsa-miR-320a-3p) and GCCUUCUCUUCCCGGUUCUUCC (SEQ ID NO:3 hsa-miR-320a-5p). In a preferred embodiment hsa-miR-320a has SEQ ID NO:2.

Most existing media that are used for human embryo culturing have a content that approximates the amount found naturally in the reproductive system of the mother in vivo.

Examples of embryos that can be cultured utilizing the methods described herein include any animal cell, but preferably include mammalian embryos. Mammalian embryos that can be used include, without limitation, bovine, equine, porcine, canine, feline, ovine, simian, lupine, murine, leporine, and more preferably include human or homo sapiens embryos. In one embodiment, the embryo is from an endangered species. In one embodiment, the embryo is from a domesticated species (e.g., cattle, horses, sheep, pigs, dogs, cats, and the like, without limitation). In another embodiment, the embryo is from a non-human primate. It should be understood that one or more of the species listed above can be specifically excluded from one or more of the methods described herein.

The medium can be any suitable medium for embryo culture, including, for example, a sequential medium or a single medium that is replenished at least once during the culture process, or a non-sequential medium or a medium that is not replenished (e.g., uninterrupted or continuous culture) during the culturing of the embryo. Existing media that are known to those of skill in the art or that are commercially available. Some non-limiting examples of existing media that can be utilized in the methods or that can be modified for use include, without limitation, Continuous Single Culture™ media (Irvine Scientific), any Global® media (LifeGlobal), G1 medium (Vitrolife), G2 medium (Vitrolife), a Human Tubal Fluid (HTF) medium, HTF medium with or without glucose or phosphate, Whitten's Medium, Ham's F-10 Medium, Sage Media, and the like, with or without protein supplementation. Further preferred media are mentioned in Table 1 of M.Tarahomi et al., Human Reproduction, pp. 1-12, 2019

In some embodiments, the embryo is transferred into a patient suffering from infertility. In some embodiments, the embryo is transferred into a patient undergoing fertility treatments. In some embodiments, the embryo is transferred into a surrogate. That is, the embryo need not be transferred back to the patient who provided the oocyte. In some embodiments, the embryo is cryopreserved. In some embodiments, a cryopreserved embryo is later transferred to a female patient. In some embodiments, the embryo is transferred into a patient suffering from recurrent implantation failure (RIF).

### EXAMPLE

### Endometrial biopsies

Biopsies were performed on hysterectomy specimens from patients that received surgery for spotting due to a niche in a caesarean section scar (n=2). Patients were pre-menopausal, had a history of proven fertility and at least one live birth, and received no hormonal treatment 3 months prior to surgery. Biopsies were performed randomly in the menstrual cycle and biopsied material was immediately suspended in Dulbecco's Modified Eagle Medium/Nutrient Mixture F-12 phenol red-free medium (DMEM/F12; L-glutamine, HEPES; Thermo Fisher Scientific, Waltham, MA, USA) supplemented with 1% penicillin/streptomycin at 37°C. Next, the biopsied material was cut into small pieces and further digested enzymatically in DMEM/F12 phenol red-free medium supplemented with >125 U/ml collagenase IV and 1% penicillin/streptomycin. The suspended material was filtered through a 76 µm sterile filter and subsequently cultured in DMEM/F12 phenol red-free medium supplemented with 10% heat-inactivated fetal calf serum (FCS; Thermo Fisher Scientific, Waltham, MA, USA) and 1% penicillin/streptomycin (C-medium). The medium was refreshed after 3 hours to discard all non-attached cells and retain the attached hESCs. Purity of hESCs cultures was assessed by immunostaining for vimentin (M0725, Agilent, CA, USA) and cytokeratin 18 (M7010, Agilent, CA, USA). Only hESCs cultures that were passaged less than 6 times were used for subsequent experiments.

### Embryo conditioned medium (ECM)

All assigned ECM was collected from regular ICSI-cycles in which routine ICSI procedures were followed and during which all embryos were handled according to local regulations and standard operating procedures. Embryos were cultured in Sage medium (Quinn's advantage cleavage medium, Cooper Surgical, USA) supplemented with 5% human serum albumin (HSA; Vitrolife, Göteborg, Sweden) from day 1-3 after fertilization. Thereafter, embryos were transferred to fresh Sage medium (Quinn's advantage blastocyst medium, Cooper Surgical, USA) supplemented with 5% HSA, on day 3 after fertilization. Embryo morphology was assessed daily by an independent laboratory technician. Droplets (20 µl) from individually cultured embryos were collected in sterile 1.5 ml collection tubes after all embryos had been removed from the culture dishes for cryopreservation on day 4. All ECM samples were collected on day 4. Empty medium droplets, in which no embryo was grown but that otherwise underwent the same procedures, from the same dishes were used as controls. Samples were snap-frozen in liquid nitrogen and stored at -80 °C.

### Migration assays

### Experimental groups

ECM was used from individually cultured embryos with high morphological quality (≥8 blastomeres, fragmentation ≤20%), low morphological quality (≤7 blastomeres, fragmentation >20%) or empty control medium in which no embryo had been cultured, but that otherwise underwent the same laboratory procedures. Per experimental n, ECM from 5 individually cultured embryos was pooled (90 µl).

### Experimental procedures

30,000 hESCs were cultured in single wells of a 48-wells plate and decidualized by adding DMEM/F12 phenol red-free medium supplemented with 10% heat-inactivated charcoal stripped FCS (Thermo Fisher Scientific, Waltham, MA, USA), 0.5 mM 8-bromoadenosine 3',5'-cyclic monophosphate (Sigma-Aldrich, Saint Louis, MO, USA), 1 µM medroxyprogesterone acetate (Sigma-Aldrich, Saint Louis, MO, USA) and 1% penicillin/streptomycin (D-medium) for 5 consecutive days. Monolayers of hESCs were scratched with a p200 pipet tip to create a migration zone. HESCs were then cultured in 90 µL of pooled ECM derived from 5 individual embryos. Cultures were overlaid with 150 µL mineral oil (Irvine Scientific, Santa Ana, CA, USA) to prevent evaporation. In case RNase was added to migration assays, we used 1 mg/ml of RNase in PBS (10109169001, Roche, Basel, Switzerland). At 0 hours and 18 hours after creation of the migration zone, phase contrast images were taken using a monochrome digital camera (Leica DFC365 FX) attached to an inverted microscope (Olympus IX71) with a U Plan FL 4x/0.13 PhL objective. The migration response of hESCs was quantified by assessing the reduction of the surface area of the migration zone by using Image J software (version 1.50i).

### miRNome PCR

### Experimental groups

ECM from individually cultured embryos of high-quality (fragmentation ≤20%; 48 samples), low-quality (fragmentation >20%; 48 samples) or empty control medium, in which no embryo had been cultured (48 samples), was used. Per experimental group, three biological replicates were performed by pooling 16 individually cultured ECM samples (320 µl pooled ECM). To ascertain that cellular fragmentation was the only difference among these experimental groups, samples from embryos with different developmental stages (ranging from 6 blastomeres to the morula stage) were evenly distributed among both experimental groups.

### Experimental procedures

All procedures for miRNome analysis were performed at Qiagen, Hilden, Germany. Total RNA was extracted using miRCURY RNA isolation kit - Biofluids (300112, Exiqon, Denmark) according to manufacturer's instructions and RNA was eluted in 50 µl RNase-free H2O. Next, RNA was polyadenylated and cDNA was synthesized according to manufacturer's instructions (miRCURY LNA RT Kit, 339340, Qiagen, Hilden, Germany). Expression of miRNAs was determined by Ready-to-Use Human panel I+II PCR, using ExiLENT SYBR® Green master mix (339322, Qiagen, Hilden, Germany). Amplification reactions were performed in a LightCycler 480 Real-Time PCR System (05015243001, Roche, Basel, Switzerland) in 384 well plates. Amplification curves were analyzed using LC software (04994884001, Roche, Basel, Switzerland), both for determination of Cq values and for melting curve analysis. Only samples with Cq<37 were included in the analysis.

### miRNA transfections

Hsa-miR mimics (339173, miRCURY LNA miRNA Mimic) and inhibitors (339121, miRCURY LNA miRNA Inhibitors) were ordered from Qiagen, Hilden, Germany. Additionally, negative control mimics (YM00479902, Negative Control miRCURY LNA miRNA Mimic) and negative control inhibitors (YI00199006, Negative control A), which both have no homology to any known miRNA or mRNA in human, were ordered from Qiagen, Hilden, Germany. The following miRNAs were labeled with a fluorescent 5'-FAM tag: hsa-miR-19a mimic + inhibitor, hsa-miR-19b mimic + inhibitor, negative control A mimic, negative control inhibitor. 30,000 decidualized or non-decidualized hESCs were cultured in single wells of a 48 wells plate and transfected with 25 nM miRNA mimic or negative control mimic. Alternatively, 30,000 hESCs were transfected with 150 nM miRNA inhibitor or negative control inhibitor. All transfections were performed in D- or C-medium by using HiPerFect Transfection Reagent according to manufacturer's instructions (301704, Qiagen, Hilden, Germany). After transfection for 24 hours, hESCs were washed with PBS and transfection efficiency was determined by calculating the percentage of fluorescently labelled hESCs from the total number of hESCs per image.

### RNA-seq

### RNA isolation

Per well of a 6-wells plate 300,000 hESCs were cultured and decidualized for 5 consecutive days, after which hESCs were transfected for 24 hours with 25 nM hsa-miR-320a mimic or 25 nM negative control mimic. Directly after, transfection medium was removed and hESCs were harvested by using a cell scraper (3010, Corning, NY, USA) and passed through a 20 gauge syringe needle. Next, total RNA was isolated using the ISOLATE II RNA Mini Kit (BIO-52072, Bioline, London, UK) according to manufacturer's instructions.

### Library construction and RNA-sequencing

Total RNA was sent to BGI Tech Solutions (Hong Kong, China) for library construction and sequencing (BGISeq500, paired end 100 bp). Twelve libraries were constructed for non-stranded pair-end cDNA sequencing on the BGISEQ-500 RNA-Seq platform. Cleaned sequence reads were mapped against the reference human Hg38 build (USC Genome Browser) using HISAT2. The counts of the aligned reads were summarized with featureCounts in the Rsubread package. Multiple mapped reads were excluded from counting. Fold changes were calculated, and differential analysis was performed with the R packages edgeR-Voom. Genes exhibiting differential expression between MIR_320A and MIR_CTRL transfected hESCs with p < 0.05 were included in further analyses.

### Statistical analysis

Mean differences among experimental groups were tested by one-way ANOVA or student's t-test. To correct for multiple testing we used Dunnett and Tukey post-hoc tests when applicable. In all cases, IBM SPSS Statistics 23 software was used. P-values <0.05 were considered statistically significant.

### Results

### The migratory effect of ECM from high-quality embryos can be abolished by RNase

We first confirmed that decidualized hESCs migration was stimulated by ECM from high-quality embryos compared to empty control medium (p=0.002, Figure 1A,B). To determine whether this stimulatory signal was caused by secreted RNA molecules, we repeated these migration assays but with the addition of RNase. We found that the addition of RNase completely abolished the pro-migratory effect of ECM from high-quality embryos on decidualized hESCs (p=0.008, Figure 1A,B).

### High- and low-quality human preimplantation embryos secrete specific subsets of miRNAs

To identify the nature of the soluble RNA molecules that stimulated migration of decidualized hESCs, we performed a PCR-array analyzing the presence of 752 human miRNAs in ECM. ECM was collected and pooled based on the morphological quality of the embryos: high-quality embryos (48 ECM droplets from individually cultured embryos; fragmentation ≤20%), low-quality embryos (48 ECM droplets from individually cultured embryos; fragmentation >20%) or empty control medium (48 droplets) (Figure 2A). From each set of 48 ECM samples of either high- or low-quality, 16 samples each were pooled resulting in three biological replicates for both high- and low-quality ECM (Figure 2A).

In total, 53 unique human miRNAs were identified in at least one biological replicate, that were not present in control medium: 20 miRNAs unique to high-quality embryos, 25 miRNAs unique to low-quality embryos, and 8 miRNAs that were overlapping between high- and low-quality embryos (Figure 2B). MiRNAs that were most frequently detected in ECM, regardless of the experimental group, were: hsa-miR-16-5p (5/6 experiments), hsa-miR-372-3p (5/6 experiments), has-miR-345-5p (4/6 experiments) and hsa-miR-21-5p (3/6 experiments) (Figure 2C). MiRNAs that were detected in 2/6 experiments were: hsa-miR-320a, hsa-miR-19b-3p, hsa-miR-19a-3p, hsa-let-7a-5p, hsa-miR-1913, hsa-miR-877-3p and hsa-miR-671-5p (Figure 2C). We then identified which miRNAs were exclusively detectable in ECM from either high- or low-quality embryos, and found that hsa-miR-320a was specific to high-quality ECM (2/3 experiments), while hsa-miR-19b-3p and hsa-miR-19a-3p were specific to low-quality ECM (2/3 experiments) (Figure 2C). All other miRNAs that were specific for either high-quality or low-quality embryos were only expressed in one biological replicate. Additional family members of the aforementioned miRNAs were not detected in any of the samples.

### Endometrial stromal cell migration is regulated by miRNA hsa-miR-320a secretion from high-quality embryos

To study the effects of miRNAs that were exclusively secreted by either high-quality or low-quality embryos on decidualized hESCs migration, we transfected hESCs with mimics or inhibitors for either hsa-mir-320a (high-quality ECM) or hsa-miR19b-3p and hsa-miR-19a-3p (low-quality ECM) (Figure 3A). Both mimics and inhibitors were successfully transfected into decidualized hESCs, as visualized by a green fluorescent signal for those miRNAs that could be designed with a fluorescently labelled 5'-FAM tag (Figure 3B). No significant difference in transfection efficiency, determined by the percentage of fluorescently labeled hESCs, was detected between negative controls and miRNA-specific mimics (68.2% vs. 57.6%, p=0.422) or inhibitors (64.7% vs. 53.1%, p=0.100) (Figure 3C).

Next, we identified which of the detected miRNAs was able to mimic ECM induced changes in decidualized hESCs migration, in the absence of ECM. To do so, we first transfected miRNAs into monolayers of decidualized hESCs in the presence of regular culture medium alone. We found that hsa-miR-320a stimulated migration of decidualized hESCs in the absence of ECM (p=0.002), while there was no effect from hsa-miR19b-3p (p=0.995) or hsa-miR-19a-3p (p=0.837), compared to transfection with negative control mimics (Figure 3D). These negative controls were designed to have no homology to any known miRNA or mRNA in human, and are therefore reflective for baseline conditions.

We then asked whether transfection with hsa-miR-320a mimics would increase the migration response of decidualized hESCs that were subsequently cultured in ECM, derived from low-quality embryos (<8 blastomeres, >20% fragmentation) or empty control medium. To do so, decidualized hESCs were first transfected with hsa-miR-320a mimics, after which migration assays were performed with ECM (Figure 4A). Indeed, decidualized hESCs that were transfected with hsa-miR-320a mimics and that were subsequently exposed to control ECM or low-quality ECM, showed an increased migration response, compared to transfection with negative control mimics (control ECM, p=0.028; low-quality ECM, p=0.040) (Figure 4B).

Next, we examined whether transfection of decidualized hESCs with hsa-miR-320a inhibitors would interfere with signals present in high-quality ECM (≥8 blastomeres, ≤20% fragmentation). First, we confirmed that high-quality ECM stimulated migration of decidualized hESCs after transfection with negative control inhibitors (p=0.034) (Figure 4C). Again, these negative controls were designed to have no homology to any known miRNA or mRNA in human, and are therefore reflective for baseline conditions. Then, after transfection with hsa-miR-320a inhibitors, we demonstrated that decidualized hESCs migration was no longer stimulated by high-quality ECM (p=0.003; compared to control p=0.732) (Figure 4C). In other words, inhibition of hsa-miR-320a completely antagonized any pro-migratory effects of high-quality ECM.

We then questioned whether decidualization was required for hESCs to respond to hsa-miR-320a. To do so, we transfected non-decidualized hESCs with mimics and inhibitors for hsa-miR-320a and repeated the migration assays during co-culture with high-quality ECM (≥8 blastomeres, ≤20% fragmentation) or empty control medium. We found that mimicking or inhibiting hsa-miR-320a function in non-decidualized hESCs did not affect the response to high-quality ECM or empty control medium (Supplementary Figure 1).

### Hsa-miR-320a may influence decidualized hESCs migration by targeting cell adhesion and cytoskeleton organization

Finally, we investigated which specific genes were targeted by embryo-derived hsa-miR-320a in hESCs. To do so, we performed RNA-sequencing on decidualized hESCs derived from 2 patients that were treated with either hsa-miR-320a mimics (n=3 per patient) or negative controls (n=3 per patient). Apart from patient variation, principle component analysis demonstrated a clear effect of hsa-miR-320a mimics on the transcriptome landscape of decidualized hESCs (Figure 5A). In total, hsa-miR-320a mimics induced differential expression of 223 transcripts. Of these transcripts, 140 were downregulated, and 83 were upregulated in decidualized hESCs treated with hsa-miR-320a mimics compared to negative controls (Figure 5B, supplementary data 1A). Next, we used gene ontology analysis to estimate which biological processes in hESCs could be affected by hsa-miR-320a (Huang, Sherman, and Lempicki 2009a, 2009b). By doing so, we identified various processes that were significantly affected by hsa-miR-320a, of which cell adhesion and regulation of cytoskeleton organization could be directly related to the observed migration response of decidualized hESCs in response to high-quality ECM (False discovery rate of < 0.05) (Table 1, supplementary data 1B).

### References

Abu-Halima, M., S. Häusler, C. Backes, T. Fehlmann, C. Staib, S. Nestel, I. Nazarenko, E. Meese, and A. Keller. 2017. 'Micro-ribonucleic acids and extracellular vesicles repertoire in the spent culture media is altered in women undergoing In Vitro Fertilization', Sci Rep, 7: 13525.
Balaban, B., D. Brison, G. Calderón, J. Catt, J. Conaghan, L. Cowan, T. Ebner, D. Gardner, T. Hardarson, K. Lundin, M. Cristina Magli, D. Mortimer, S. Mortimer, S. Munné, D. Royere, L. Scott, J. Smitz, A. Thornhill, J. van Blerkom, and E. Van den Abbeel. 2011. 'The Istanbul consensus workshop on embryo assessment: proceedings of an expert meeting', Hum Reprod, 26: 1270-83.
Berkhout, R. P., C. B. Lambalk, J. Huirne, V. Mijatovic, S. Repping, G. Hamer, and S. Mastenbroek. 2018. 'High-quality human preimplantation embryos actively influence endometrial stromal cell migration', J Assist Reprod Genet, 35: 659-67.
Bidarimath, M., K. Khalaj, R. T. Kridli, F. W. Kan, M. Koti, and C. Tayade. 2017. 'Extracellular vesicle mediated intercellular communication at the porcine maternal-fetal interface: A new paradigm for conceptus-endometrial cross-talk', Sci Rep, 7: 40476.
Calin, G. A., and C. M. Croce. 2006. 'MicroRNA signatures in human cancers', Nat Rev Cancer, 6: 857-66.
Capalbo, A., F. M. Ubaldi, D. Cimadomo, L. Noli, Y. Khalaf, A. Farcomeni, D. Ilic, and L. Rienzi. 2016. 'MicroRNAs in spent blastocyst culture medium are derived from trophectoderm cells and can be explored for human embryo reproductive competence assessment', Fertil Steril, 105: 225-35 e1-3.
Cha, J., X. Sun, and S. K. Dey. 2012. 'Mechanisms of implantation: strategies for successful pregnancy', Nat Med, 18: 1754-67.
Cuman, C., M. Van Sinderen, M. P. Gantier, K. Rainczuk, K. Sorby, L. Rombauts, T. Osianlis, and E. Dimitriadis. 2015. 'Human Blastocyst Secreted microRNA Regulate Endometrial Epithelial Cell Adhesion', EBioMedicine, 2: 1528-35.
Ebner, T., M. Moser, M. Sommergruber, and G. Tews. 2003. 'Selection based on morphological assessment of oocytes and embryos at different stages of preimplantation development: a review', Hum Reprod Update, 9: 251-62.
Esquela-Kerscher, A., and F. J. Slack. 2006. 'Oncomirs - microRNAs with a role in cancer', Nat Rev Cancer, 6: 259-69.
Feng, R., Q. Sang, Y. Zhu, W. Fu, M. Liu, Y. Xu, H. Shi, Y. Xu, R. Qu, R. Chai, R. Shao, L. Jin, L. He, X. Sun, and L. Wang. 2015. 'MiRNA-320 in the human follicular fluid is associated with embryo quality in vivo and affects mouse embryonic development in vitro', Sci Rep, 5: 8689.
Fletcher, D. A., and R. D. Mullins. 2010. 'Cell mechanics and the cytoskeleton', Nature, 463: 485-92.
Fritz, R., C. Jain, and D. R. Armant. 2014. 'Cell signaling in trophoblast-uterine communication', Int J Dev Biol, 58: 261-71.
Galliano, D., and A. Pellicer. 2014. 'MicroRNA and implantation', Fertil Steril, 101: 1531-44.
Gao, T., M. Deng, and Q. Wang. 2018. 'MiRNA-320a inhibits trophoblast cell invasion by targeting estrogen-related receptor-gamma', J Obstet Gynaecol Res, 44: 756-63.
Gellersen, B., and J. J. Brosens. 2014. 'Cyclic decidualization of the human endometrium in reproductive health and failure', Endocr Rev, 35: 851-905.
Grewal, S., J. G. Carver, A. J. Ridley, and H. J. Mardon. 2008. 'Implantation of the human embryo requires Rac1-dependent endometrial stromal cell migration', Proc Natl Acad Sci U S A, 105: 16189-94.
Guo, T., Y. Feng, Q. Liu, X. Yang, T. Jiang, Y. Chen, and Q. Zhang. 2014. 'MicroRNA-320a suppresses in GBM patients and modulates glioma cell functions by targeting IGF-1R', Tumour Biol, 35: 11269-75.
Ha, M., and V. N. Kim. 2014. 'Regulation of microRNA biogenesis', Nat Rev Mol Cell Biol, 15: 509-24.
Haouzi, D., H. Dechaud, S. Assou, C. Monzo, J. de Vos, and S. Hamamah. 2011. 'Transcriptome analysis reveals dialogues between human trophectoderm and endometrial cells during the implantation period', Hum Reprod, 26: 1440-9.
He, L., and G. J. Hannon. 2004. 'MicroRNAs: small RNAs with a big role in gene regulation', Nat Rev Genet, 5: 522-31.
Huang, da W, B. T. Sherman, and R. A. Lempicki. 2009a. 'Bioinformatics enrichment tools: paths toward the comprehensive functional analysis of large gene lists', Nucleic Acids Res, 37: 1-13.
---. 2009b. 'Systematic and integrative analysis of large gene lists using DAVID bioinformatics resources', Nat Protoc, 4: 44-57.
Kropp, J., S. M. Salih, and H. Khatib. 2014. 'Expression of microRNAs in bovine and human pre-implantation embryo culture media', Front Genet, 5: 91.
Li, H., L. Yu, J. Liu, X. Bian, C. Shi, C. Sun, X. Zhou, Y. Wen, D. Hua, S. Zhao, L. Ren, T. An, W. Luo, Q. Wang, and S. Yu. 2017. 'miR-320a functions as a suppressor for gliomas by targeting SND1 and β-catenin, and predicts the prognosis of patients', Oncotarget, 8: 19723-37.
Liu, J., Z. Song, C. Feng, Y. Lu, Y. Zhou, Y. Lin, and C. Dong. 2017. 'The long non-coding RNA SUMO1P3 facilitates breast cancer progression by negatively regulating miR-320a', Am J Transl Res, 9: 5594-602.
Lou, W., J. Liu, Y. Gao, G. Zhong, D. Chen, J. Shen, C. Bao, L. Xu, J. Pan, J. Cheng, B. Ding, and W. Fan. 2017. 'MicroRNAs in cancer metastasis and angiogenesis', Oncotarget, 8: 115787-802.
Lv, G., M. Wu, M. Wang, X. Jiang, J. Du, K. Zhang, D. Li, N. Ma, Y. Peng, L. Wang, L. Zhou, W. Zhao, Y. Jiao, X. Gao, and Z. Hu. 2017. 'miR-320a regulates high mobility group box 1 expression and inhibits invasion and metastasis in hepatocellular carcinoma', Liver Int, 37: 1354-64.
Lv, Q., J. X. Hu, Y. J. Li, N. Xie, D. D. Song, W. Zhao, Y. F. Yan, B. S. Li, P. Y. Wang, and S. Y. Xie. 2017. 'MiR-320a effectively suppresses lung adenocarcinoma cell proliferation and metastasis by regulating STAT3 signals', Cancer Biol Ther, 18: 142-51.
Macklon, Geraedts, Fauser. 2002. 'Conception to ongoing pregnancy The 'black box' of early pregnancy loss'.
Macklon, N. S., and J. J. Brosens. 2014. 'The human endometrium as a sensor of embryo quality', Biol Reprod, 91: 98.
Marco, A., K. Hooks, and S. Griffiths-Jones. 2012. 'Evolution and function of the extended miR-2 microRNA family', RNA Biol, 9: 242-8.
McCallie, B., W. B. Schoolcraft, and M. G. Katz-Jaffe. 2010. 'Aberration of blastocyst microRNA expression is associated with human infertility', Fertil Steril, 93: 2374-82.
Niakan, K. K., J. Han, R. A. Pedersen, C. Simon, and R. A. Pera. 2012. 'Human pre-implantation embryo development', Development, 139: 829-41.
Peter Durairaj, R. R., A. Aberkane, L. Polanski, Y. Maruyama, M. Baumgarten, E. S. Lucas, S. Quenby, J. K. Chan, N. Raine-Fenning, J. J. Brosens, H. Van de Velde, and Y. H. Lee. 2017. 'Deregulation of the endometrial stromal cell secretome precedes embryo implantation failure', Mol Hum Reprod.
Puissant, F., M. Van Rysselberge, P. Barlow, J. Deweze, and F. Leroy. 1987. 'Embryo scoring as a prognostic tool in IVF treatment', Hum Reprod, 2: 705-8.
Qi, X., J. Li, C. Zhou, C. Lv, and M. Tian. 2014. 'MicroRNA-320a inhibits cell proliferation, migration and invasion by targeting BMI-1 in nasopharyngeal carcinoma', FEBS Lett, 588: 3732-8.
Quenby, S., and J. J. Brosens. 2013. 'Human implantation: a tale of mutual maternal and fetal attraction', Biol Reprod, 88: 81.
Rhee, J. K., S. Y. Shin, and B. T. Zhang. 2013. 'Construction of microRNA functional families by a mixture model of position weight matrices', PeerJ, 1: e199.
Rosenbluth, E. M., D. N. Shelton, L. M. Wells, A. E. Sparks, and B. J. Van Voorhis. 2014. 'Human embryos secrete microRNAs into culture media--a potential biomarker for implantation', Fertil Steril, 101: 1493-500.
van Loendersloot, L., M. van Wely, F. van der Veen, P. Bossuyt, and S. Repping. 2014. 'Selection of embryos for transfer in IVF: ranking embryos based on their implantation potential using morphological scoring', Reprod Biomed Online, 29: 222-30.
Van Royen, E., K. Mangelschots, D. De Neubourg, M. Valkenburg, M. Van de Meerssche, G. Ryckaert, W. Eestermans, and J. Gerris. 1999. 'Characterization of a top quality embryo, a step towards single-embryo transfer', Hum Reprod, 14: 2345-9.
Weimar, C. H., A. Kavelaars, J. J. Brosens, B. Gellersen, J. M. de Vreeden-Elbertse, C. J. Heijnen, and N. S. Macklon. 2012. 'Endometrial stromal cells of women with recurrent miscarriage fail to discriminate between high- and low-quality human embryos', PLoS One, 7: e41424.
Wilcox, A. J., C. R. Weinberg, J. F. O'Connor, D. D. Baird, J. P. Schlatterer, R. E. Canfield, E. G. Armstrong, and B. C. Nisula. 1988. 'Incidence of early loss of pregnancy', N Engl J Med, 319: 189-94.
Xishan, Z., L. Ziying, D. Jing, and L. Gang. 2015. 'MicroRNA-320a acts as a tumor suppressor by targeting BCR/ABL oncogene in chronic myeloid leukemia', Sci Rep, 5: 12460.
Yao, J., L. H. Liang, Y. Zhang, J. Ding, Q. Tian, J. J. Li, and X. H. He. 2012. 'GNAI1 Suppresses Tumor Cell Migration and Invasion and is Post-Transcriptionally Regulated by Mir-320a/c/d in Hepatocellular Carcinoma', Cancer Biol Med, 9: 234-41.
Yu, J., J. G. Wang, L. Zhang, H. P. Yang, L. Wang, D. Ding, Q. Chen, W. L. Yang, K. H. Ren, D. M. Zhou, Q. Zou, Y. T. Jin, and X. P. Liu. 2016. 'MicroRNA-320a inhibits breast cancer metastasis by targeting metadherin', Oncotarget, 7: 38612-25.
Zhang, Y., X. He, Y. Liu, Y. Ye, H. Zhang, P. He, Q. Zhang, L. Dong, and J. Dong. 2012. 'microRNA-320a inhibits tumor invasion by targeting neuropilin 1 and is associated with liver metastasis in colorectal cancer', Oncol Rep, 27: 685-94.
Zhang, Z., X. Li, W. Sun, S. Yue, J. Yang, J. Li, B. Ma, J. Wang, X. Yang, M. Pu, B. Ruan, G. Zhao, Q. Huang, L. Wang, K. Tao, and K. Dou. 2017. 'Loss of exosomal miR-320a from cancer-associated fibroblasts contributes to HCC proliferation and metastasis', Cancer Lett, 397: 33-42.
Zhao, H., T. Dong, H. Zhou, L. Wang, A. Huang, B. Feng, Y. Quan, R. Jin, W. Zhang, J. Sun, D. Zhang, and M. Zheng. 2014. 'miR-320a suppresses colorectal cancer progression by targeting Rac1', Carcinogenesis, 35: 886-95.
Zhao, W., Q. Sun, Z. Yu, S. Mao, Y. Jin, J. Li, Z. Jiang, Y. Zhang, M. Chen, P. Chen, D. Chen, H. Xu, and S. Ding. 2018. 'MiR-320a-3p/ELF3 axis regulates cell metastasis and invasion in non-small cell lung cancer via PI3K/Akt pathway', Gene, 670: 31-37.
Zinaman, M. J., E. D. Clegg, C. C. Brown, J. O'Connor, and S. G. Selevan. 1996. 'Estimates of human fertility and pregnancy loss', Fertil Steril, 65: 503-9.

## Claims

1. mir320a or a mimic thereof for use in an assisted reproduction treatment.

2. mir320a or a mimic thereof for use according to claim 1, wherein said treatment is in a human.

3. An embryo transfer medium suitable for use in a human comprising mir320a or a mimic thereof.

4. The embryo transfer medium according to claim 3, comprising a medium approved for use in human IVF treatments.

5. The embryo transfer medium according to claim 3 or 4, comprising 10-50 nM, more preferably about 25 nM mir320a or miRNA mimic.

6. The embryo transfer medium according to claim 3-5 for use in an assisted reproduction treatment.


SEQUENCE LISTING

<110> Academisch Medisch Centrum

<120> EMBRYO TRANSFER MEDIUM

<130> 2018-093EP-PR

<160> 3

<170> BiSSAP 1.3.6

<210> 1
<211> 72
<212> RNA
<213> Artificial Sequence

<220>
<223> pre-miR320a

<400> 1
cuccccuccg ccuucucuuc ccgguucuuc ccggagucgg gaaaagcugg guugagaggg 60

cgaaaaagga ug 72

<210> 2
<211> 22
<212> RNA
<213> Artificial Sequence

<220>
<223> hsa-miR-320a-3p

<400> 2
aaaagcuggg uugagagggc ga 22

<210> 3
<211> 22
<212> RNA
<213> Artificial Sequence

<220>
<223> hsa-miR-320a-5p

<400> 3
gccuucucuu cccgguucuu cc 22